(19) Europäisches Patentamt
European Patent Office
Office européen des brevets

(11) Veröffentlichungsnummer: **0 075 737**
**A1**

(12) **EUROPÄISCHE PATENTANMELDUNG**

(21) Anmeldenummer: 82107925.8

(22) Anmeldetag: 28.08.82

(51) Int. Cl.³: C 07 D 277/06
C 07 D 279/06, C 07 D 279/12
C 07 D 205/04, C 07 D 207/16
C 07 D 211/60, C 07 D 211/62
A 01 N 43/00

(30) Priorität: 19.09.81 DE 3137376

(43) Veröffentlichungstag der Anmeldung: 06.04.83 Patentblatt 83/14

(84) Benannte Vertragsstaaten: AT BE CH DE FR GB IT LI NL

(71) Anmelder: Degussa Aktiengesellschaft
Weissfrauenstrasse 9
D-6000 Frankfurt am Main 1(DE)

(72) Erfinder: Drauz, Karlhein, Dr.
Flurstrasse 5
D-6463 Freigericht(DE)

(72) Erfinder: Kleemann, Axel, Dr.
Greifenhagenstrasse
D-6450 Hanau 9(DE)

(54) **N-Chlorcarbonyl-aminocarbonsäureester, Verfahren zu deren Herstellung und deren Verwendung.**

(57) Gegenstand der Erfindung sind N-Chlorcarbonylaminocarbonsäureester der allgemeinen Formel

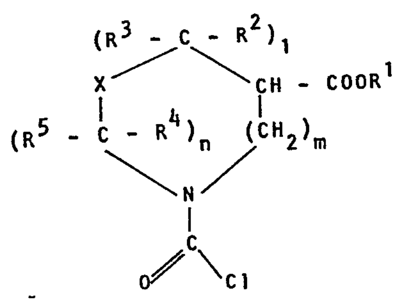

(1)

in der X, $R^1$, $R^2$, $R^3$, $R^4$, $R^5$, 1, m und n bestimmte näher definierte Bedeutungen haben. Sie werden dadurch hergestellt, dass mittels Phosgen die Chlorcarbonylgruppe in den entsprechenden Aminocarbonsäureester eingeführt wird, und können zur Herstellung von Wirkstoffen des Hydantointyps bzw. des Harnstofftyps, insbesondere für Pflanzenschutzmittel, verwendet werden.

EP 0 075 737 A1

Degussa Aktiengesellschaft
Weissfrauenstrasse 9, 6000 Frankfurt 1

# N-Chlorcarbonyl-aminocarbonsäureester, Verfahren zu deren Herstellung und deren Verwendung

## Beschreibung:

Gegenstand der Erfindung sind N-Chlorcarbonyl-amino-carbonsäureester der allgemeinen Formel

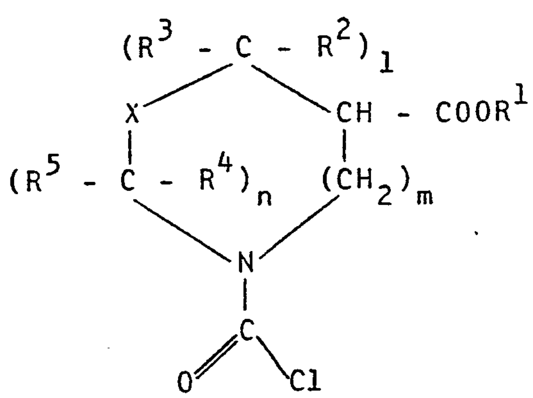

(I),

in der X - $CH_2$ - oder - S -, $R^1$ einen geradkettigen oder verzweigten Alkylrest mit bis zu 4 Kohlenstoffatomen, $R^2$, $R^3$ und $R^4$ unabhängig voneinander jeweils Wasserstoff oder einen Methylrest, $R^5$ Wasserstoff, einen unsubstituierten oder durch gegen Phosgen beständige Substituenten substituierten Alkylrest oder einen unsubstituierten oder durch gegen Phosgen beständige Substituenten substituierten aromatischen oder heteroaromatischen Rest, oder $R^4$ und $R^5$ zusammen einen Alkylenrest mit 4 oder 5 Kohlenstoffatomen und l, m und n unabhängig voneinander jeweils 0, 1 oder 2 bedeuten mit der Massgabe, dass die Summe aus l, m und n minimal 1 und maximal 3 beträgt und dass für den Fall l = 2 $R^2$ und $R^3$ nur die Bedeutung Wasserstoff haben dürfen, sowie ein Verfahren zu deren Herstellung, welches dadurch gekennzeichnet ist, dass man einen Aminocarbonsäureester der allgemeinen Formel

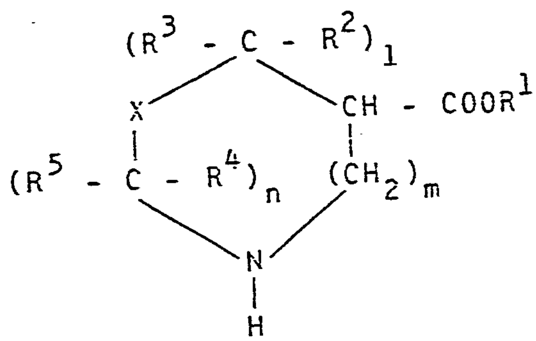

(II),

in der X, $R^1$, $R^2$, $R^3$, $R^4$, $R^5$, l, m und n die bereits angegebene Bedeutung haben, oder dessen Hydrohalogenid in einem gegenüber Phosgen inerten Lösungsmittel bei einer Temperatur zwischen - 30 und + 100°C mit der 1- bis 10-fachen molaren Menge an Phosgen zur Reaktion bringt.

Die N-Chlorcarbonyl-aminocarbonsäureester der allgemeinen Formel (I) können auf einfache Weise zu an sich bekannten Wirkstoffen weiterverarbeitet werden. So bilden sie z.B. mit primären Aminen Wirkstoffe des Hydantointyps oder mit sekundären Aminen Wirkstoffe des Harnstofftyps.

Ein weiterer Gegenstand der Erfindung ist daher die Verwendung der N-Chlorcarbonyl-aminocarbonsäureester der allgemeinen Formel (I) zur Herstellung von Wirkstoffen, insbesondere für Pflanzenschutzmittel.

Von besonderer Wichtigkeit sind unter den N-Chlorcarbonyl-aminocarbonsäureestern der allgemeinen Formel (I) unter anderem diejenigen, für welche n = 1 ist und der Rest $R^5$ eine der folgenden Bedeutungen hat:

Wasserstoff;
unsubstituierter, geradkettiger oder verzweigter Alkylrest, insbesondere mit 1 bis 16 Kohlenstoffatomen, wie Methyl, Ethyl, n-Propyl- i-Propyl, n-Butyl, n-Pentyl, n-Hexyl, n-Heptyl, n-Octyl, n-Nonyl, n-Decyl, n-Undecyl, n-Tridecyl, n-Pentadecyl oder n-Hexadecyl;
mit gegen Phosgen beständigen Substituenten substituierter Alkylrest, wie Phenylmethyl, (1'-Phenyl)-ethyl, (2'-Methylmercapto)-ethyl oder (2'-Ethylmercapto)-ethyl;

unsubstituierter aromatischer oder heteroaromatischer Rest, wie Phenyl, Naphthyl, 2'-Furyl, 2'-Thienyl oder 3'-Thienyl;
mit gegen Phosgen beständigen Substituenten substituierter aromatischer oder heteroaromatischer Rest, wie 2'-Fluorphenyl, 3'-Fluorphenyl, 4'-Fluorphenyl, 2'-Chlorphenyl , 3'-Chlorphenyl, 4'-Chlorphenyl, 3',4'-Dichlorphenyl, 3',5'-Dichlorphenyl, 2',4'-Dichlorphenyl, 2',6'-Dichlorphenyl, 2',5'-Dichlorphenyl, 2'-Nitrophenyl, 3'-Nitrophenyl, 4'-Nitrophenyl, 2'-Methoxyphenyl, 3'-Methoxyphenyl, 4'-Methoxyphenyl, 4'-Ethoxyphenyl, 4'-Propoxyphenyl, 4'-Butoxyphenyl, 3',4'-Dimethoxyphenyl, Piperonyl (3',4'-Methylendioxophenyl), 3'-Phenoxyphenyl, 4'-Phenoxyphenyl, 2'-Methylphenyl, 3'-Methylphenyl, 4'-Methylphenyl, 4'-tert.Butylphenyl, 4'-n-Pentylphenyl, 2'-Trifluoromethylphenyl, 3'-Trifluoromethylphenyl, 4'-Trifluoromethylphenyl, 5'-Chlor-2'-thienyl, 5'-Brom-2'-thienyl, 4'-Brom-2'-thienyl, 5'-Nitro-2'-thienyl oder 5'-Nitro-2'-furyl.

Die Umsetzung der Aminocarbonsäureester der allgemeinen Formel (II) mit dem Phosgen wird vorzugsweise bei einer Temperatur zwischen - 20 und + 80°C vorgenommen.

Beispiele für gegenüber Phosgen inerte Lösungsmittel sind Kohlenwasserstoffe, wie Hexan oder Toluol; Chlorkohlenwasserstoffe, wie Methylenchlorid, Chloroform oder 1,2-Dichlorethan; Ether, wie Methyl-tert.butylether oder Dioxan; oder Carbonsäureester, wie Essigsäureethylester, Essigsäurepropylester oder Propionsäuremethylester.

Sofern die Aminocarbonsäureester der allgemeinen Formel (II) ein Asymmetriezentrum aufweisen, können

sie als die reinen Enantiomeren oder als Racemat eingesetzt werden. Bei der Umsetzung mit dem Phosgen bleibt unter den angegebenen Reaktionsbedingungen die optische Konfiguration erhalten.

Die Aminocarbonsäureester der allgemeinen Formel (II) können nach bekannten Veresterungsmethoden aus den ihnen zugrundeliegenden freien Aminocarbonsäuren hergestellt werden. Die freien Aminocarbonsäuren sind zum Teil natürlich vorkommende Aminocarbonsäuren, wie Azetidincarbonsäure oder Prolin, zum Teil werden sie kommerziell hergestellt, wie Pipecolinsäure oder Isonipecotinsäure. Soweit es sich um unnatürliche Aminocarbonsäuren handelt, können sie aus natürlich vorkommenden oder käuflichen Aminocarbonsäuren, Aminocarbonsäurederivaten oder Aminocarbonsäurevorstufen durch einfache Reaktionen dargestellt werden, z.B. durch Kondensation geeigneter nichtcyclischer Aminocarbonsäuren mit geeigneten Aldehyden oder Ketonen.

Die Umsetzung der Aminocarbonsäureester der allgemeinen Formel (II) mit dem Phosgen erfolgt zweckmässigerweise so, dass das Phosgen in dem gewählten Lösungsmittel gelöst und der Aminocarbonsäureester zudosiert wird. Die Aminocarbonsäureester können in reiner Form oder in Form der rohen Reaktionsprodukte aus der Dehydrohalogenierung der entsprechenden Hydrohalogenide mit Ammoniak eingesetzt werden.

Alternativ ist es auch möglich, die Hydrohalogenide der Aminocarbonsäureester der allgemeinen Formel (II) direkt einzusetzen. In diesem Falle ist es zweckmässig, das Hydrohalogenid in dem gewählten Lösungsmittel aufzulösen bzw. zu suspendieren und das Phosgen zu der Lösung bzw. Suspension zuzugeben.

Die Umsetzung mit dem Phosgen erfordert je nach eingesetztem Aminocarbonsäureester der allgemeinen Formel (II) eine Reaktionszeit zwischen 1 und 50 Stunden. In jedem Falle muss die Reaktion solange fortgeführt werden, bis anfänglich vorhandene Suspensionen oder zwischenzeitlich auftretende Niederschläge vollständig in Lösung gegangen sind.

Die Aufarbeitung erfolgt in der Weise, dass zunächst das Lösungsmittel und das überschüssige Phosgen, gegebenenfalls unter vermindertem Druck, abgezogen werden. Diese wiedergewonnenen Materialien können ohne weiteres erneut eingesetzt werden. Sofern der gebildete N-Chlorcarbonyl-aminocarbonsäureester flüchtig ist, kann dann der verbleibende Rückstand zur weiteren Reinigung, gegebenenfalls unter vermindertem Druck, destilliert werden.

Hat der gebildete N-Chlorcarbonyl-aminocarbonsäureester ein höheres Molekulargewicht - im allgemeinen ist das der Fall, wenn der die Chlorcarbonylgruppe tragende Ring 6-Ringglieder aufweist und/oder aromatische oder heteroaromatische Substituenten trägt - lässt er sich häufig nicht mehr unzersetzt destillieren. In diesen Fällen kann eine weitere Reinigung dadurch erfolgen, dass der nach dem Abziehen des Lösungsmittels und des überschüssigen Phosgens verbleibende Rückstand in frischem Lösungsmittel aufgenommen und einer Aktivkohleklärung unterworfen wird. Nach Filtration wird dann das Filtrat bei vermindertem Druck vom Lösungsmittel befreit und man erhält ein Öl, das gegebenenfalls kristallisiert.

Ist der gebildete N-Chlorcarbonyl-aminocarbonsäureester optisch aktiv, dann kann seine optische Reinheit z.B. dadurch überprüft werden, dass man eine Probe der

BAD ORIGINAL

Hydrolyse unterwirft, die freigesetzte Aminosäure isoliert und ihren Drehwert bestimmt. Man findet, dass die ursprüngliche optische Konfiguration unverändert erhalten geblieben ist.

Die N-Chlorcarbonyl-aminocarbonsäureester der allgemeinen Formel (I) lassen sich mit den verschiedenartigsten primären und sekundären Aminen zu an sich, z.B. aus der britischen Patentschrift 1 503 244, bekannten Wirkstoffen des Hydantointyps bzw. des Harnstofftyps umsetzen. Diese Umsetzung wird in Gegenwart eines Säureacceptors, z.B. von Pyridin oder Triethylamin, in mindestens äquimolaren Mengen vorgenommen. Sie kann in Abwesenheit von zusätzlichen Lösungsmitteln oder in einem geeigneten Lösungsmittel, z.B. in Dioxan oder Toluol, erfolgen. Die Reaktionstemperatur kann dabei zwischen etwa 20°C und der Rückflusstemperatur des Reaktionsgemisches liegen. Die gewünschten Hydantoin- bzw. Harnstoff-Derivate werden innerhalb relativ kurzer Reaktionszeiten von z.B. 4 bis 5 Stunden in hohen Ausbeuten erhalten.

Die Erfindung wird durch die nachfolgenden Beispiele näher erläutert:

Beispiel 1:

1,2 Mol (181,9 g) L-Azetidincarbonsäure(2)-methylester·HCl werden in 800 ml Chlorform suspendiert und unter Rühren und Kühlen bei 0°C mit Ammoniakgas gesättigt. Das ausgefallene Ammoniumchlorid wird abgesaugt, das Filtrat im Vakuum eingedampft. Der Rückstand wir bei 0°C unter Rühren innerhalb von 2 Stunden zu einer Lösung von 200 ml Phosgen in 400 ml Chloroform zugetropft, die Reaktionsmischung anschließend 12 Stunden bei Raumtemperatur gerührt. Nach Abdampfen des Lösungsmittels und des überschüssigen Phosgens wird im Vakuum destilliert. Es gehen bei 100 - 105°C und einem Druck von 1,3 mbar 137,5 g (64,5 %) L-N-Chlorcarbonyl-azetidin-carbonsäuremethylester über.

| $C_6H_8ClNO_3$ | Berechnet (%) | | Gefunden (%) |
|---|---|---|---|
| (177,59) | C | 40,58 | 39,82 |
| | H | 4,54 | 4,49 |
| | Cl | 19,96 | 20,03 |
| | N | 7,89 | 8,23 |

$^1$H-NMR ($CDCl_3$): δ = 4,85 (mc, 1H) CH - $COOCH_3$,
4,33 (mc, 2H) $CH_2$- NCOCl,
3,85 (s, 3H) $COOCH_3$,
3,3 - 1,9 ppm (m, 2H) $CH_2$ - CH - $COOCH_3$.

IR (flüssig kapillar ) : 1740 cm $^{-1}$.

Beispiel 2:

5,0 Mol (828,1 g) L-Prolinmethylester·HCl werden mit Ammoniak gemäß Beispiel 1 in den freien Ester überführt. Der rohe Prolinmethylester wird bei 0°C unter Rühren innerhalb von 45 Minuten zu einer Lösung von 1000 ml Phosgen in 2000 ml Chloroform zugetropft. Es wird eine weitere Stunde bei dieser Temperatur und weitere 12 Stunden bei Raumtemperatur nachgerührt. Nach Abdampfen des Chloroforms und des überschüssigen Phosgens wird der Rückstand im Vakuum destilliert. Man erhält 896,1 g (93,5 %) L-N-Chlorcarbonyl-prolinmethylester mit einem Siedepunkt von 90 - 95°C bei 0,012 mbar.

| $C_7H_{10}Cl\ NO_3$ | | Berechnet (%) | Gefunden (%) |
|---|---|---|---|
| (191,62) | C | 43,88 | 44,16 |
| | H | 5,26 | 5,46 |
| | Cl | 18,50 | 17,93 |
| | N | 7,31 | 7,38 |

$^1$H-NMR ($CDCl_3$): $\delta$ = 4,53 (mc, 1H) -<u>CH</u> -$COOCH_3$,
3,97 - 3,53 (m, 2H) -<u>$CH_2$</u> -N -COCl,
3,87; 3,83 (s, 3H) -COO<u>$CH_3$</u>,
2,19 ppm (mc, 4H) -<u>$CH_2$</u> -<u>$CH_2$</u> - .

IR (flüssig kapillar): 1735 $cm^{-1}$.

Beispiel 3:

1,0 Mol (165,6 g) L-Prolinmethylester·HCl werden in 500 ml Chloroform gelöst, und 200 ml Phosgen zu der auf +5°C abgekühlten Lösung unter Rühren innerhalb von 30 Minuten zugetropft.
Man rührt 12 Stunden bei +5°C nach, weitere 5 Stunden bei Raumtempertaur und anschließend noch 24 Stunden bei +40°C. Durch entsprechende Kühlung wird das überschüssige Phosgen im Reaktionsraum gehalten.
Nach Aufarbeitung gemäß Beispiel 2 und Destillation erhält man 168,0 g (87,7 %) L-N-Chlorcarbonyl-prolinmethylester mit einem Siedepunkt von 90 - 95°C bei 0,013 mbar.

Beispiel 4:

19,1 g (0,1 Mol) N-Chlorcarbonyl-prolinmethylester, 11,0 g Triethylamin und 10,7 g (0,1 Mol) 4-Methylanilin werden in 100 ml Dioxan 4 Stunden unter starkem Rühren auf 90°C erhitzt. Nach Abkühlen wird die Reaktionsmischung unter starkem Rühren auf 500 ml Eiswasser gegossen. Der dabei entstehende feinkristalline Niederschlag wird abgesaugt und bei 50°C und 13 mbar getrocknet.
Man erhält 21,4 g (93 %) 1,5-Trimethylen-3-(4'-fluorphenyl)-hydantoin mit einem Schmelzpunkt von 161 - 162°C.

Beispiel 5:

19,1 g (0,1 Mol) N-Chlorcabonyl-prolinmethylester, 8,2 g Pyridin und 11,1 g (0,1 Mol) 4-Fluoranilin werden in 200 ml Toluol 5 Stunden unter Rückfluß erhitzt. Nach Reaktionsende wird das Toluol am Rotationsverdampfer abgezogen und der Rückstand unter kräftigem Rühren auf 500 ml Eiswasser gegossen. Nach Absaugen und Trocknen bei 50°C und 13 mbar erhält man 20,6 g (88 %) 1,5-Trimethylen-3-(4-fluorphenyl)-hydantoin mit einem Schmelzpunkt von 143 - 146°C.

Beispiel 6:

19,1 g (0,1 Mol) N-Chlorcarbonyl-prolinmethylester, 8,5 g Pyridin und 16,2 g (0,1 Mol) 3,4-Dichloranilin werden gemäß Beispiel 5 umgesetzt. Nach Aufarbeitung erhält man 24,7 g (86,5 %) 1,5-Trimethylen-3-(3',4'-dichlorphenyl)hydantoin mit einem Schmelzpunkt von 141 - 145°C.

Beispiel 7:

19,1 g (0,1 Mol) N-Chlorcarbonyl-prolinmethylester und 16,2 g (0,1 Mol) 3,5-Dichloranilin werden in 100 ml Pyridin 5 Stunden unter kräftigem Rühren unter Rückfluß erhitzt. Nach Entfernen des Pyridins unter vermindertem Druck und Gießen des Rückstandes auf 500 ml Eiswasser gemäß Beispiel 5 erhält man 25,3 g (89 %) 1,5-Trimethylen-3-(3',5'-dichchlorphenyl)hydantoin.

Beispiel 8:

1,0 Mol (179,65 g) L-Prolinethylester·HCl werden gemäß Beispiel 2 nach Überführung in den freien Ester mit Phosgen umgesetzt. Nach Aufarbeitung erhält man 196,4 g (95,5 %) L-N-Chlorcarbonyl-prolin-ethylester mit einem Siedepunkt von 90°C bei 0,0013 mbar.

| $C_8H_{12}Cl\ NO_3$ | | Berechnet (%) | Gefunden (%) |
|---|---|---|---|
| (205,64) | C | 46,73 | 46,80 |
| | H | 5,88 | 6,03 |
| | Cl | 17,24 | 17,20 |
| | N | 6,81 | 7,21 |

$^1$H-NMR ($CDCl_3$): δ = 4,47 (mc, 1H) CH -$COOC_2H_5$,
4,27; 423 (q, I=7Hz; 2H) $COOCH_2CH_3$,
3,75 (mc, 2H) $CH_2$-NCOCl,
2,13 (mc, 4H) -$CH_2$-$CH_2$-,
1,33 ppm (t, I=7Hz; 3H) $COOCH_2CH_3$.

IR (flüssig kapillar): 1740 $cm^{-1}$.

Beispiel 9:

0,5 Mol (71,6 g) D,L-Pipecolinsäuremethylester werden bei -10°C innerhalb von 30 Minuten zu einer Lösung

von 100 ml Phosgen in 400 ml Chloroform zugetropft. Die Reaktionsmischung wird anschließend 12 Stunden bei Raumtemperatur nachgerührt. Nach Abdampfen des Lösungsmittels und des überschüssigen Phosgens wird der erhaltene Rückstand einer Aktivkohleklärung unterworfen, nach Filtration und Abdampfen des Lösungsmittels erhält man 100,9 g (98,1 %) D,L-N-Chlorcarbonyl-pipecolinsäuremethylester als Öl.

| $C_8H_{12}Cl\ NO_3$ | | Berechnet (%) | Gefunden (%) |
|---|---|---|---|
| (205,64) | C | 46,72 | 46,90 |
| | H | 5,88 | 6,08 |
| | Cl | 17,24 | 17,15 |
| | N | 6,81 | 7,17 |

IR (flüssig kapillar ): 1735 $cm^{-1}$.

Beispiel 10:

20,6 g (0,1 Mol) N-Chlorcarbonyl-pipecolinsäuremethylester, 11,1 g (0,1 Mol) 3-Fluoranilin werden in 150 ml Pyridin 4 Stunden unter kräftigen Rühren auf Siedetemperatur erhitzt. Nach Reaktionsende wird das überschüssige Lösungsmittel unter verminderten Druck abgedampft und der verbleibende Rückstand unter kräftigem Rühren auf 500 ml Eiswasser gegeossen. Die dabei ausfallende kristalline Substanz wird abgesaugt, mit Eiswasser nachgewaschen und bei 50°C/13 mbar im Trockenschrank bis zur Gewichtskonstanz getrocknet.

Man erhält so 20,8 g (84 %) 1,5-Tetramethylen-3-(3'-fluorphenyl)-hydantoin mit einem Schmelzpunkt von 116 - 119°C.

Beispiel 11:

20,6 g (0,1 Mol) N-Chlorcarbonyl-pipecolinsäuremethylester und 10,7 g (0,1 Mol) 4-Methylanilin werden gemäß Beispiel 10 umgesetzt.
Nach Aufarbeitung erhält man 21,6 g (88,5 %) 1,5-Tetramethyl-3-(4'-methylphenyl)-hydantoin mit einem Schmelzpunkt von 185 - 188 °C.

Beispiel 12:

0,77 Mol (138,4 g) Isonipecotinsäuremethylester.HCl werden mit Ammoniak gemäß Beispiel 1 in den freien Ester überführt. Dieser wird dann innerhalb einer Stunde zu einer Lösung von 150 ml Phosgen in 400 ml Chloroform zugetropft. Die Reaktionstemperatur beträgt dabei -5°C, anschließend wird 12 Stunden bei Raumtemperatur nachgerührt. Nach Aufarbeitung und Destillation erhält man 157,8 g (99,6 %) des N-Chlorcarbonyl-isonipecotinsäuremethylester mit einem Siedepunkt von 120°C/0,65 mbar.

| $C_8H_{12}Cl\ NO_3$ | | Berechnet (%) | Gefunden (%) |
|---|---|---|---|
| (205,64) | C | 46,72 | 46,61 |
| | H | 5,88 | 5,80 |
| | Cl | 17,24 | 17,35 |
| | N | 6,81 | 6,84 |

IR (KBr - Preßling): 1720 $cm^{-1}$.

$^1H$-NMR ($CDCl_3$): $\delta$ = 4,35; 4,11 (m,2H) ($\underline{CH_2}$)$_2$-NCOCl,
3,75 (s,3H) $COO\underline{CH_3}$,
3,6 - 2,9 (m,2H) ($\underline{CH_2}$)$_2$-NCOCl,
2,65 (mc,1H) $\underline{CH}$-$COOCH_3$,
2,0 ppm (mc,4H) 2-$\underline{CH_2}$-.

**Beispiel 13:**

2,0 Mol (367,3 g) D,L-Thiazolidincarbonsäuremethylester(4)·HCl werden mit Ammoniak gemäß Beispiel 1 in den freien Ester überführt. Dieser wird dann bei -10°C innerhalb von 30 Minuten unter kräftigem Rühren zu einer Lösung von 400 ml Phosgen in 800 ml Chloroform zugetropft. Anschließend läßt man 15 Stunden bei Raumtemperatur stehen. Nach Aufarbeitung und Destillation erhält man 403,0 g (96,1 %) D,L-N-Chlorcarbonylthiazolidincarbonsäuremethylester(4) mit einem Siedepunkt von 118 - 120°C/0,65 mbar.

| | | Berechnet (%) | Gefunden (%) |
|---|---|---|---|
| $C_6H_8ClNO_3S$ | | | |
| (209,65) | C | 34,37 | 34,20 |
| | H | 3,84 | 3,81 |
| | N | 6,68 | 6,81 |
| | Cl | 16,91 | 16,46 |
| | S | 15,30 | 15,25 |

IR (KBr - Preßling): 1730 $cm^{-1}$.

$^1H$-NMR ($CDCl_3$): $\delta$ =5,2 - 4,4 (m,3H) CH-$COOCH_3$, S-$CH_2$-N,
3,85; 3,80 (s,3H) $COOCH_3$,
3,6 - 3,1 ppm (m,2H) $CH_2$-CH-$COOCH_3$.

Beispiel 14:

0,46 Mol (74,2 g) D,L-2-Methylthiazolidincarbonsäuremethylester(4) werden gemäß Beispiel 9 mit 100 ml Phosgen in 400 ml Chlororform umgesetzt.
Nach Aufarbeitung und Destillation erhält man 84,5 g (82,1 %) D,L-3-Chlorcarbonyl-2-methylthiazolidincarbonsäuremethylester(4) mit einem Siedepunkt von 98 - 100°C/ 0,39 mbar.

| $C_7H_{10}CLNO_3S$ | | Berechnet (%) | Gefunden (%) |
|---|---|---|---|
| (223,68) | C | 37,59 | 37,52 |
| | H | 4,51 | 4,60 |
| | Cl | 15,85 | 15,61 |
| | N | 6,26 | 6,31 |
| | S | 14,33 | 14,17 |

IR (flüssig kapillar ): 1735 $cm^{-1}$.

$^1H$-NMR ($CDCl_3$): $\delta$ = 5,65 - 4,65 (m,2H) CH-$COOCH_3$ und S-$CH_2$-N,
3,82 (s,3H) $COOCH_3$,
3,6 - 3,2 (m,2H) $CH_2$-CH-$COOCH_3$,
1,65 ppm (mc,3H) CH-$CH_3$.

Beispiel 15:

1,0 Mol (211,7 g) D,L-2,2-Dimethylthiazolidincarbonsäuremethylester(4)·HCl werden mit Ammoniak gemäß Beispiel 1 in den freien Ester überführt. Dieser wird dann bei -5°C unter Rühren zu einer Lösung von 200 ml Phosgen in 350 ml Chloroform zugetropft. Anschließend wird 12 Stunden bei Raumtemperatur gerührt. Die Aufarbeitung erfolgt gemäß Beispiel 9.
Man erhält 230,1 g (96,8 %) D,L-3-Chlor-carbonyl-2-dimethylthiazolidincarbonsäuremethylester als farbloses Öl.

IR (flüssig kapillar): 1740 $cm^{-1}$.

$^{1}$H-NMR ($CDCl_3$): $\delta$ = 5,2 (mc,1H) <u>CH</u>-$COOCH_3$,
4,85 (s,3H) COO<u>$CH_3$</u>,
3,37 (mc,2H) S-<u>$CH_2$</u>-N,
1,95; 1,85 ppm (s,6H) C(<u>$CH_3$</u>$)_2$.

Beispiel 16:

1,5 Mol (386,6 g) frisch hergestellter D,L-2(p-Chlorphenyl)-thiazolidincarbonsäuremethylester(4) wird in 300 ml Chloroform gelöst und bei 0°C zu einer Lösung von 300 ml Phosgen in 500 ml Chloroform zugetropft. Man rührt 2 Stunden bei 0°C nach, läßt 12 Stunden bei Raumtemperatur stehen und erwärmt die Reaktionsmischung anschließend noch 3 Stunden auf 50°C. Die dann klare Lösung wird unter vermindertem Druck eingedampft, der Rückstand in 1 l Chlororform aufgenommen, mit Aktivkohle 1 Stunde unter Rückfluß erhitzt, abfiltriert, eingedampft und durch Zugabe von 200 ml Methyl-tert.butylether aufgefällt.
Man erhält 390,5 g (81,3 %) D,L-3-Chlorcarbonyl-2-(p-chlorphenyl)-thiazolidincarbonsäuremethylester(4) mit einem Schmelzpunkt von 118 - 121°C.

| $C_{12}H_{11}Cl_2NO_3S$ | | Berechnet (%) | Gefunden (%) |
|---|---|---|---|
| (320,19) | C | 45,01 | 45,27 |
| | H | 3,46 | 3,50 |
| | Cl | 22,15 | 22,36 |
| | N | 4,38 | 4,81 |
| | S | 10,19 | 10,02 |

IR (KBr - Preßling) 1745 (Schulter), 1725 $cm^{-1}$.

$^{1}H$-NMR ($CDCl_3$): $\delta$ = 7,8 - 7,15 (m, 4H) Aromatenprotonen,
6,23; 6,08 (s, 1H) S-<u>CH</u>-N,
5,05 (mc, 1H) <u>CH</u>-$COOCH_3$,
3,83 (s, 3H) $COO\underline{CH_3}$,
3,4 ppm (mc, 2H) $\underline{CH_2}$-CH-$COOCH_3$.

**Beispiel 17:**

1,0 Mol (211,7 g) D-5,5-Dimethylthiazolidincarbonsäuremethylester·HCl werden gemäß Beispiel 16 dehydrologeniert und phosgeniert. Die Vakuumdestillation ergibt 202,0 g (85 %) D-3-Chlorcarbonyl-5,5-dimethyl-thiazolidincarbonsäureester(4) mit einem Siedepunkt von 117°C/0,65 mbar.

IR (flüssig kapillar): 1735 $cm^{-1}$.

$^1$H-NMR ($CDCl_3$): $\delta$ = 4,85 (S,1H) <u>CH</u>-$COOCH_3$,
4,60 (mc,2H) S-<u>$CH_2$</u>-N,
3,78 (S,3H) -COO<u>$CH_3$</u>,
1,6; 1,3 ppm (S,6H)-C(<u>$CH_3$</u>)$_2$.

Beispiel 18:

1,0 Mol (239,8 g) D-2,2,5,5-Tetramethylthiazolidin-carbonsäuremethylester(4) werden gemäß Beispiel 18 dehydrohalogeniert und phosgeniert. Nach Aufarbeitung gemäß Beispiel 16 erhält man 256,8 g (96,6 %) D-3-Chlor-carbonyl-2,2,5,5-tetramethylthiazolidincarbonsäure-methylester(4) als Öl.

| $C_{10}H_{16}ClNO_3S$ | | Berechnet (%) | Gefunden (%) |
|---|---|---|---|
| (265,76) | C | 45,19 | 45,51 |
| | H | 6,07 | 6,24 |
| | Cl | 13,34 | 13,05 |
| | N | 5,27 | 5,34 |
| | S | 12,07 | 12,52 |

IR (flüssig kapillar): 1735 $cm^{-1}$.

$^1$H-NMR ($CDCl_3$): $\delta$ = 4,75 (s,1H) <u>CH</u>-$COOCH_3$,
3,80 (s,3H) $COO\underline{CH_3}$,
1,95 (s,6H) -N-$C(\underline{CH_3})_2$-S-,
1,75; 1,4 ppm (s,6H) $C(\underline{CH_3})_2$.

## Beispiel 19:

0,2 Mol (36,7 g) D,L-Thiazolidincarbonsäuremethylester (2)·HCl werden gemäß Beispiel 14 dehydrohalogeniert und phosgeniert. Nach Aufarbeitung und Destillation erhält man 39,8 g (94,9 %) D,L-3-Chlorcarbonylthiazolidincarbonsäuremethylester-(2) mit einem Siedepunkt von 105 - 110°C/0,0065 mbar.

| $C_6H_8ClNO_3S$ | | Berechnet (%) | Gefunden (%) |
|---|---|---|---|
| (209,65) | C | 34,37 | 34,87 |
| | H | 3,84 | 3,90 |
| | N | 6,68 | 6,51 |
| | Cl | 16,91 | 16,52 |
| | S | 15,30 | 15,15 |

IR (flüssig kapillar): 1735 $cm^{-1}$.

## Beispiel 20:

1,0 Mol (197,7 g) D,L-Tetrahydrothiazin(1,3)-carbonsäuremethylester-(4).HCl werden gemäß Beispiel 1

dehydrohalogeniert. Der rohe D,L-Tetrahydrothiazin-(1,3)-carbonsäuremethylester wird bei -5°C innerhalb von 45 Minuten zu einer Lösung von 180 ml Phosgen in 500 ml Chloroform zugetropft, 12 Stunden bei Raumtemperatur und weitere 3 Stunden bei 45°C gerührt. Die jetzt klare Lösung wird eingedampft und der Rückstand gemäß Beispiel 16 aufgearbeitet.
Man erhält 223,5 g (~ 100 %) D,L-3-Chlorcarbonyl-tetrahydrothiazin(1,3)-carbonsäuremethylester(4).

IR (flüssig kapillar): 1735 $cm^{-1}$.

$^1$H-NMR ($CDCl_3$): $\delta$ = 5,3 - 4,1 (m,3H) <u>CH</u>-$COOCH_3$ und S-<u>$CH_2$</u>-N,
3,85 (s,3H) COO<u>$CH_3$</u>,
3,15 - 1,8 ppm (m,4H)-<u>$CH_2$</u>-<u>$CH_2$</u>.

Beispiel 21:

1,0 Mol (197,7 g) D,L-Tetrahydrothiazin(1,4)-carbonsäuremethylester-(5)·HCl werden gemäß Beispiel 21 dehydrohalogeniert und phosgeniert. Nach Aufarbeitung gemäß Beispiel 16 erhält man 221,9 g (99,2 %) D,L-4-Chlorcarbonyl-tetrahydrothiazin(1,4)-carbonsäuremethylester(5).

IR (flüssig kapillar): 1730 $cm^{-1}$.

| | | Berechnet (%) | Gefunden (%) |
|---|---|---|---|
| $C_7H_{10}ClNO_3S$ (223,68) | C | 37,59 | 37,21 |
| | H | 4,51 | 4,36 |
| | Cl | 15,86 | 15,65 |
| | N | 6,26 | 6,50 |
| | S | 14,33 | 14,01 |

Degussa Aktiengesellschaft
Weissfrauenstrasse 9, 6000 Frankfurt 1

N-Chlorcarbonyl-aminocarbonsäureester, Verfahren zu deren Herstellung und deren Verwendung

Patentansprüche:

1. N-Chlorcarbonyl-aminocarbonsäureester der allgemeinen Formel

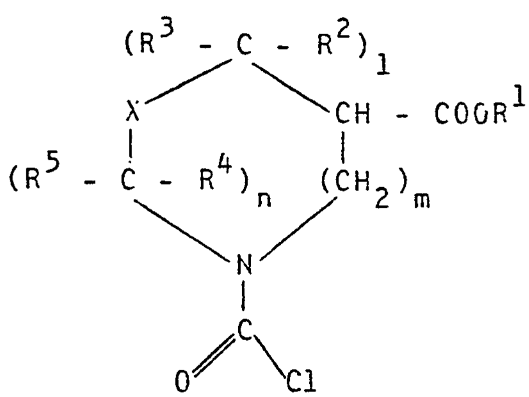

(I),

in der X - $CH_2$ - oder - S -, $R^1$ einen geradkettigen oder verzweigten Alkylrest mit bis zu 4 Kohlenstoffatomen, $R^2$, $R^3$ und $R^4$ unabhängig voneinander jeweils Wasserstoff oder einen Methylrest, $R^5$ Wasserstoff, einen unsubstituierten oder durch gegen Phosgen beständige Substituenten substituierten Alkylrest oder einen unsubstituierten oder durch gegen Phosgen beständige Substituenten substituierten aromatischen oder heteroaromatischen Rest, oder $R^4$ und $R^5$ zusammen einen Alkylenrest mit 4 bis 5 Kohlenstoffatomen und l, m und n unabhängig voneinander jeweils 0, 1 oder 2 bedeuten mit der Massgabe, dass die Summe aus l, m und n minimal 1 und maximal 3 beträgt und dass für den Fall l = 2 $R^2$ und $R^3$ nur die Bedeutung Wasserstoff haben dürfen.

2. Verfahren zur Herstellung der N-Chlorcarbonyl-aminocarbonsäureester der allgemeinen Formel (I), dadurch gekennzeichnet, dass man einen Aminocarbonsäureester der allgemeinen Formel

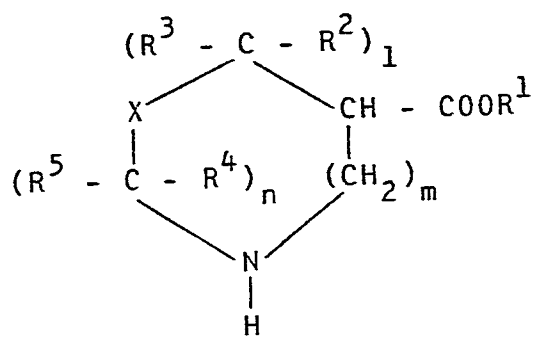

(II),

in der X, $R^1$, $R^2$, $R^3$, $R^4$, $R^5$, l, m und n die bereits angegebene Bedeutung haben, oder dessen Hydrohalogenid in einem gegen Phosgen inerten Lösungsmittel bei einer Temperatur zwischen -30 und +100°C mit der 1- bis 10-fachen molaren Menge an Phosgen zur Reaktion bringt.

3. Verwendung der N-Chlorcarbonyl-aminocarbonsäureester der allgemeinen Formel (I) zur Herstellung von Wirkstoffen, insbesondere für Pflanzenschutzmittel.

Europäisches Patentamt

# EUROPÄISCHER RECHERCHENBERICHT

0075737
Nummer der Anmeldung

EP 82 10 7925

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| | --- | | C 07 D 277/06<br>C 07 D 279/06<br>C 07 D 279/12<br>C 07 D 205/04<br>C 07 D 207/16<br>C 07 D 211/60<br>C 07 D 211/62<br>A 01 N 43/00 |
| A | CHEMICAL ABSTRACTS, Band 87, Nr. 7, 15. August 1977, Zusammenfassung Nr. 53377f, Seite 484, Columbus Ohio (US) & JP - A - 77 19 685 (TOYAMA CHEMICAL CO., LTD.) (15.02.1977)<br>--- | | |
| A | CHEMICAL ABSTRACTS, Band 84, Nr. 15, 12. April 1976, Zusammenfassung Nr. 105576e, Seite 579, Columbus Ohio (US) & JP - A - 75 126 690 (TOYAMA CHEMICAL CO., LTD.) (04.10.1975)<br>--- | | |
| A | US-A-4 138 242 (GODDARD)<br>--- | | RECHERCHIERTE SACHGEBIETE (Int. Cl. 3) |
| A | DE-A-2 221 770 (BAYER)<br>----- | | C 07 D 277/06<br>C 07 D 279/06<br>C 07 D 279/12<br>C 07 D 205/04<br>C 07 D 207/16<br>C 07 D 211/60<br>C 07 D 211/62<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 05-01-1983 | DE BUYSER I.A.F. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503. 03.82